# EUROPEAN PATENT SPECIFICATION

(11) **EP 4 298 996 B1**
(45) Date of publication and mention of the grant of the patent: **13.08.2025**
(21) Application number: 23382601.5
(22) Date of filing: 16.06.2023
(51) Int. Cl.: A61B 5/00, A61B 5/024, A61B 5/318

(54) **NON-INVASIVE CARDIAC SENSING SYSTEM**
NICHTINVASIVES HERZMESSSYSTEM
SYSTÈME DE DÉTECTION CARDIAQUE NON INVASIF

(30) Priority: 01.07.2022 ES 202230600
(43) Date of publication of application: 03.01.2024
(73) Proprietor: Arrhythmia Network Technology, S.L., 28224 Pozuelo de Alarcón (Madrid) (ES)
(72) Inventor:
(74) Representative: De la Fuente Fernandez, Dionisio

(56) References cited:
- EP-A1- 3 760 107
- US-A1- 2017 347 894
- US-A1- 2018 303 434
- US-A1- 2019 133 516
- US-A1- 2020 029 828
- US-A1- 2021 045 647

## Description

### FIELD OF THE INVENTION

The present invention reveals a non-invasive cardiac sensor system that allows the automation of heart failure measurements through Al (artificial intelligence) applied to clinical analysis, in such a way that it allows warning and predicting of relevant changes that prevent hospital admissions. The system incorporates a device that allows reading cardiac signals. These signals are sent via Bluetooth to a mobile application, where they are packaged and through an internet connection they go to a server where they will be fully processed to be consulted.

### BACKGROUND OF THE INVENTION

Cardiovascular (CV) disease is a leading cause of morbidity, mortality, and burden on the healthcare system worldwide, with approximately 7 million cases of heart failure and many more cases of myocardial infarction in the United States alone. Acute and chronic cardiovascular (CV) conditions reduce quality of life and life expectancy. Various treatment modalities have been developed for Cardiovascular (CV) disease, ranging from pharmaceuticals to mechanical devices and finally transplantation.

Heart failure (HF) is considered the main cause of hospitalization in patients over 65 years of age and of unscheduled hospital readmissions, and its estimated cost is estimated at around 2,500 million euros per year, which represents 3.8 % of global health spending. Different entities call for improvement and increased coordination between levels of care, as well as comprehensive follow-up after hospitalization, not only in the stage of suspected diagnosis. Lifetime costs for heart failure patients have been estimated at $126,819 per patient.

There are many types of temporary cardiac assist devices with varying degrees of support and invasiveness, from Intra-Aortic Balloon Pump (IABP) to Extracorporeal Membrane Oxygenation (ECMO) devices to surgically implanted Left Ventricular Assist devices (LVADs). These devices typically reside outside the ventricle or bypass the ventricle, and do not work in parallel with or directly support cardiac function. Nor do they provide clinicians with quantifiable metrics that can guide the level of cardiac support that is required for any given patient. Some ventricular assist devices are inserted percutaneously into the heart.

**The** amount of support (egg, volumetric flow rate of blood delivered by the pumping device) and/or duration of support required by each patient may vary.

Patent application GB1601593.5 reveal a vital signs monitor comprising: a housing; a power supply; a plurality of measurement modules within the housing, each module being operable to measure and monitor at least one respective vital sign; one or more inputs for connection to sensors applied to a patient and arranged to supply sensor signals to respective measurement modules; and a user display. **The** vital signs monitor may also comprise a user input device, the user input device being operable to allow a user to input data, patient details, control parameter, operating parameters, or the like, to the monitor. **The** vital signs monitor may be a portable vital signs monitor. The vital signs monitor may be a hand-held monitor. The vital signs monitor may be ergonomically shaped with respect to a user's hand. The vital signs monitor may include one or more apparatus or user attachment devices; the user attachment devices being configured such that the monitor may attachable to an apparatus or a user. The user attachment device may be a strap. The strap may be a hand strap. The strap may be configured such that it may be wrapped around the hand of a user when the user is holding the monitor. The strap may be a wrist strap or a torso strap. The housing may be of a size suitable to be carried by a user. The housing may be of a size suitable to be carried in one hand by a user.

Patent application PCT/IB2014/064377 reveals a cardiac monitoring system, whose objective is to provide a non-invasive cardiac function monitoring solution that includes a gyroscope that uses the Coriolis effect, configured to be applied in a position on a chest of a subject and in this position to obtain an angular ballistograph signal indicative of twisting recoil motion, which occurs in the subject's thorax in response to cardiovascular twist in the subject's thorax; signal processing means configured to generate from the angular ballistograph signal measured values indicative of the angular velocity of the subject's heart, indicative of the functioning of the subject's heart.

Document US 2020/029828 discloses a system comprising a measurement device comprising a first LED sensor to extract pulse photoplethysmography (PPG) on a finger, a second electroacoustic sensor that has a bell for heart sounds (PCG) and a third sensor to process an electrocardiogram with two electrodes (ECG).

According to the present invention, there is provided a non-invasive cardiac sensor system characterized by that it comprises a measurement device that allows reading cardiac signals and has 3 non-invasive sensors in charge of collecting user data: a first LED sensor to extract pulse photoplethysmography (PPG) on a finger, a second electroacoustic sensor that has a bell for heart sounds (PCG) and a third sensor to process an electrocardiogram with two electrodes (ECG), and inside; a battery rechargeable; a microcontroller; means for Bluetooth communication and LED elements to give information to the user; a mobile device that stores the data through an application (APP), where the application arranged on the mobile is adapted to present presents two types of users: a master user and a plurality of users, who interact with the measurement device daily through the mobile device of each of them with a user panel with a basic interface that incorporates a button to link the mobile device to the measurement device, previously linked by the master user through his username and a second button that allows data to be captured once the mobile device and measurement device are linked, where the master user programs notifications to the patient/user accounts, with a reminder in Pop-Up style when the time for measurements is approaching and programs depending on the status of the patients, the number of necessary daily measures; and a server, where once the data from the measurement device is collected and recorded, it is processed for viewing and subsequent consultation and there they are processed for analysis and stored in the users' database.

The presentinvention improves what has been revealed in the technique, since it makes it possible to predict and monitor heart failure effectively, without the need to use invasive procedures, in addition, it allows continuous control by the medical professional.

### DESCRIPTION OF THE DRAWINGS

To complement the description that is being made and in order to help a better understanding of the characteristics of the invention, according to a preferred example of its practical implementation, a set of drawings is attached as an integral part of said description, where, with an illustrative and non-limiting nature, the following has been represented:

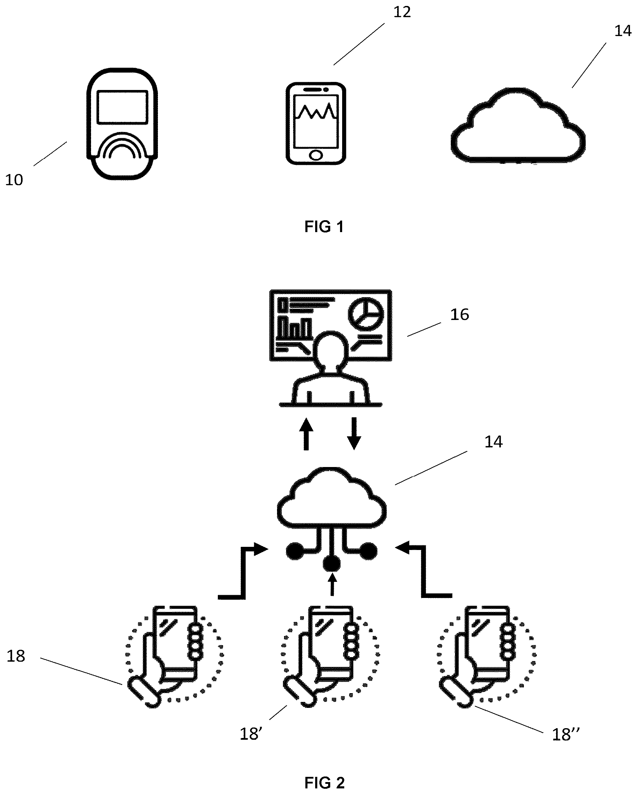

**FIG 1****.-** General view of the non-invasive cardiac sensorization system, where the device (10) is responsible for extracting the necessary data from each signal and sending it via Bluetooth to an application on the mobile (12), where it is packaged and through Internet will go to a server (14) where once the data is registered, it is processed for easy viewing and can be consulted by an expert.
**FIG 2****.-** View of the system for a plurality of users (18), (18'), (18") who interact with the device (10) daily through the application (APP) provided on the mobile (12) of each one of them or when the master user (16) indicates it.

### PREFERRED EMBODIMENT OF THE INVENTION

The present invention shows a non-invasive cardiac sensor system that allows the automation of measurements by means of Al (artificial intelligence) applied to the analysis, in such a way that it allows warning and predicting of relevant changes that avoid hospital admissions. The system incorporates a device (10) that allows reading cardiac signals. This device (10) has 3 non-invasive sensors responsible for collecting user data. A first LED sensor to extract the pulse photoplethysmography (PPG) on a finger, a second electroacoustic sensor that has a bell for heart sounds (PCG) and a third sensor to process an electrocardiogram with two electrodes (ECG), both with contact dry, it has a rechargeable battery, a microcontroller, means for Bluetooth communication and LED elements to provide information to the user.

Pulse Photoplethysmography (PPG) works with a colored LED that emits light that, when it comes into contact with the index finger or earlobe, reflects a certain amount of light. The blood flow causes the amount of reflected light to change accordingly. to the heartbeat.

Auscultation of the heart requires excellent hearing and the ability to distinguish subtle differences in pitch and duration. Low-pitched sounds are best heard with the bell. Very little pressure should be applied when using the hood. Excess pressure converts the underlying skin into a diaphragm and eliminates very low pitched sounds.

The device (10) has a button for its initiation, indicating with an LED when it has started, the equipment does not start recording data until it has been linked externally to a mobile device (12) that stores this data through an application (APP), in particular a mobile, Tablet or similar. Once linked, it begins to send, via Bluetooth communication, the data with a specific string for subsequent analysis in the application and on the server (14).

The device (10) is placed on the user's chest, making contact with the skin, both the two dry electrodes for the electrocardiogram (ECG) and the bell for heart sounds (PCG). With the same holding position of the device (10), a finger is placed on top of the LED sensor for the pulse photoplethysmography (PPG) reading. The reading time is estimated by the application (APP) previously incorporated in the mobile device (12) that has been linked to the device (10).

The device (10) can be charged by means of a 5V transformer with a USB-C port with LED warning to indicate the state of the charge (charging or charged). The device (10) incorporates the corresponding circuitry for the operation of all the chips, sensors, regulators and a previous stage of amplification and filtering for the audio signal to record PCG heart sounds.

The device (10) is in charge of extracting the necessary data from each signal and sending it via Bluetooth to an application on the mobile phone (12), where it is packaged and via the Internet will go to a server (14) where, once the data is registered, it is They are processed for easy visualization and can be consulted by an expert. The final result, after collecting the signals and treating them for analysis, corresponds to the location of some characteristic points that help in the early diagnosis of heart failure.

The mobile application (APP) arranged on the mobile (12) has two types of users. The master user (16) who would be, for example, a doctor and a plurality of users (18), (18'), (18") who would be the people who interact with the device (10) daily through the mobile (12) of each one of them or when the master user (16) indicates it.

The master user (16) has a list of his patients/users (18), (18'), (18"), where he can register and cancel equipment, associate the devices (10) with a specific patient and observe individually both the registered signals, the studies of each signal and obtain an evolution in time. Also, the master user (16) schedules alerts to the patient/user accounts (18), (18'), (18"), with a reminder (Pop-Up style) when the time to perform the measurements, as its programming, depending on the state of the patients (18), (18'), (18"), the number of measurements needed per day, etc.

The patients or users (18), (18'), (18") have user credentials in the application (APP) of their mobile (12), with a user panel with a basic interface where they have a button to link the mobile device (12) to the measurement device (10), previously linked by the master user (16) through his username and once the mobile (12) and device (10) are linked, using a second button they begin to capture the data. Once the second button is pressed, a countdown appears, before reading data, so that the users (18), (18'), (18") can accommodate the device (10) and for a few seconds of data acquisition, it will stop automatically.

Once the capture is considered good, the device (10) will send the file with all the points acquired to a server (14) and the application will be closed. Once the data reaches the server (14), there they are processed for analysis, where the characteristic points are searched for, the pertinent calculations are made and they are stored in the user database (18), (18'), (18"), corresponding. To be loaded when the master user (16) needs to see the evolution of one of her patients (18), (18'), (18'), or delve deeper into the specific analysis of one day.

The present invention enables heart failure to be monitored effectively. Management of heart failure leads to a reduction in total hospitalizations for heart failure and a reduction in 30-day readmissions for all causes.

In addition, the combination of multi-sensors is effective. The multi-sensor index and alert algorithm provide a sensitive and timely predictor of impending decompensation of HF.

## Claims

1. Non-invasive cardiac sensor system comprising a measurement device (10) that allows reading cardiac signals and has 3 non-invasive sensors in charge of collecting user data: a first LED sensor to extract pulse photoplethysmography (PPG) on a finger, a second electroacoustic sensor that has a bell for heart sounds (PCG) and a third sensor to process an electrocardiogram with two electrodes (ECG), and inside; a battery rechargeable; a microcontroller; means for Bluetooth communication and LED elements to give information to the user; a mobile device (12) that stores the data through an application (APP),where the application arranged on the mobile device presents two types of users: a master user (16) and a plurality of users (18), (18'), (18") who interact with the measurement device (10) daily through the mobile device (12) of each of them with a user panel with a basic interface that incorporates a button to link the mobile device (12) to the measurement device (10), previously linked by the master user (16) through his username and a second button that allows data to be captured once the mobile device (12) and measurement device (10) are linked, where the master user (16) programs notifications to the patient/user accounts (18), (18') , (18"), with a reminder in Pop-Up style when the time for measurements is approaching and programs depending on the status of the patients (18), (18'), (18"), the number of necessary daily measures; and a server (14), where once the data from the measurement device (10) is collected and recorded, it is processed for viewing and subsequent consultation and there they are processed for analysis and stored in the users' database (18), (18'), (18").

2. Non-invasive cardiac sensor system according to claim 1, **characterized in that** the mwasurement device (10) is placed on the user's chest, making contact with the skin, both the two dry electrodes for the electrocardiogram (ECG) and the bell for heart sounds (PCG).

3. Non-invasive cardiac sensor system according to claim 1, **characterized in that** the measurement device (10) can be charged by means of a 5V transformer with a USB-C port with LED warning to indicate the state of the charge.

4. Non-invasive cardiac sensor system according to claim 1, **characterized in that** once the mobile device (12) and the measurement device (10) are linked, they begin to capture the data by means of a second button.

## Patentansprüche

1. Nichtinvasives Herzsensorsystem, das eine Messvorrichtung (10) umfasst, die das Lesen von Herzsignalen erlaubt und 3 nichtinvasive Sensoren aufweist, die für das Sammeln von Benutzerdaten zuständig sind: einen ersten LED-Sensor, um die Puls-Photoplethysmographie (PPG) an einem Finger zu extrahieren, einen zweiten elektroakustischen Sensor, der eine Glocke für Herztöne (PKG) aufweist, und einen dritten Sensor, um ein Elektrokardiogramm mit zwei Elektroden (EKG) zu verarbeiten, und innen; eine wiederaufladbare Batterie; einen Mikrocontroller; Mittel zur Bluetooth-Kommunikation und LED-Elemente zur Bereitstellung von Informationen an den Benutzer; eine mobile Vorrichtung (12), die die Daten über eine Anwendung (APP) speichert, wobei die auf der mobilen Vorrichtung angeordnete Anwendung zwei Arten von Benutzern darstellt: einen Hauptbenutzer (16) und eine Vielzahl von Benutzern (18), (18'), (18"), die täglich mit der Messvorrichtung (10) über ihre jeweilige mobile Vorrichtung (12) mit einem Bedienpanel mit einer Basisschnittstelle, enthaltend eine Schaltfläche zur Verknüpfung der mobilen Vorrichtung (12) mit der Messvorrichtung (10), die zuvor von dem Hauptbenutzer (16) über seinen Benutzernamen verknüpft wurde, und eine zweite Schaltfläche, die die Datenerfassung erlaubt, sobald die mobile Vorrichtung (12) und die Messvorrichtung (10) verknüpft sind, interagieren, wobei der Hauptbenutzer (16) Benachrichtigungen an die Patienten-/Benutzerkonten (18), (18'), (18") programmiert, mit einer Erinnerung im Pop-Up-Stil, wenn die Zeit für Messungen näher rückt, und abhängig vom Status der Patienten (18), (18'), (18") die Anzahl der notwendigen täglichen Messungen programmiert; und einen Server (14), auf dem die Daten, sobald sie von der Messvorrichtung (10) gesammelt und aufgezeichnet worden sind, zur Ansicht und anschließenden Abfrage aufbereitet werden und dort zur Analyse verarbeitet und in der Datenbank (18), (18'), (18") der Benutzer gespeichert werden.

2. Nichtinvasives Herzsensorsystem nach Anspruch 1, **dadurch gekennzeichnet, dass** die Messvorrichtung (10) auf der Brust des Benutzers platziert wird, wobei sowohl die zwei Trockenelektroden für das Elektrokardiogramm (EKG) als auch die Glocke für die Herztöne (PKG) mit der Haut in Kontakt kommen.

3. Nichtinvasives Herzsensorsystem nach Anspruch 1, **dadurch gekennzeichnet, dass** die Messvorrichtung (10) mittels eines 5V-Transformators mit einem USB-C-Anschluss mit LED-Warnung geladen werden kann, um den Ladezustand anzuzeigen.

4. Nichtinvasives Herzsensorsystem nach Anspruch 1, **dadurch gekennzeichnet, dass**, sobald die mobile Vorrichtung (12) und die Messvorrichtung (10) miteinander verknüpft sind, sie mittels einer zweiten Schaltfläche mit der Erfassung der Daten beginnen.

## Revendications

1. Système de capteur cardiaque non invasif comprenant un dispositif de mesure (10) qui permet de lire des signaux cardiaques et a 3 capteurs non invasifs chargés de recueillir des données d'utilisateur : un premier capteur à DEL pour extraire la photopléthysmographie du pouls (PPG) sur un doigt, un deuxième capteur électroacoustique qui a une cloche pour les bruits du cœur (PCG) et un troisième capteur pour traiter un électrocardiogramme avec deux électrodes (ECG), et à l'intérieur ; une batterie rechargeable ; un microcontrôleur ; des moyens de communication Bluetooth et des éléments à DEL pour donner des informations à l'utilisateur ; un dispositif mobile (12) qui stocke les données par l'intermédiaire d'une application (APP), où l'application agencée sur le dispositif mobile présente deux types d'utilisateurs : un utilisateur principal (16) et une pluralité d'utilisateurs (18), (18'), (18") qui interagissent avec le dispositif de mesure (10) quotidiennement par l'intermédiaire du dispositif mobile (12) de chacun d'entre eux avec un panneau d'utilisateur avec une interface de base qui incorpore un bouton pour relier le dispositif mobile (12) au dispositif de mesure (10), préalablement relié par l'utilisateur principal (16) par l'intermédiaire de son nom d'utilisateur et un second bouton qui permet à des données d'être capturées une fois que le dispositif mobile (12) et le dispositif de mesure (10) sont reliés, où l'utilisateur principal (16) programme des notifications aux comptes patient/utilisateur (18), (18'), (18"), avec un rappel de style fenêtre contextuelle lorsque l'heure des mesures approche et programme en fonction du statut des patients (18), (18'), (18"), le nombre de mesures quotidiennes nécessaires ; et un serveur (14), où une fois que les données du dispositif de mesure (10) sont collectées et enregistrées, elles sont traitées en vue d'une visualisation et d'une consultation ultérieure, et elles sont traitées en vue d'une analyse et stockées dans la base de données des utilisateurs (18), (18'), (18").

2. Système de capteur cardiaque non invasif selon la revendication 1, **caractérisé en ce que** le dispositif de mesure (10) est placé sur la poitrine de l'utilisateur, entrant en contact avec la peau, à la fois les deux électrodes sèches pour l'électrocardiogramme (ECG) et la cloche pour les bruits du cœur (PCG).

3. Système de capteur cardiaque non invasif selon la revendication 1, **caractérisé en ce que** le dispositif de mesure (10) peut être chargé au moyen d'un transformateur 5V avec un port USB-C avec un avertissement à DEL pour indiquer l'état de la charge.

4. Système de capteur cardiaque non invasif selon la revendication 1, **caractérisé en ce qu'**une fois que le dispositif mobile (12) et le dispositif de mesure (10) sont reliés, ils commencent à capturer les données à l'aide d'un second bouton.
